# EUROPÄISCHE PATENTSCHRIFT

(11) **EP 1 485 063 B1**
(45) Veröffentlichungstag und Bekanntmachung des Hinweises auf die Patenterteilung: **10.05.2006**
(21) Anmeldenummer: 03709753.2
(22) Anmeldetag: 06.03.2003
(51) Int. Cl.: A61K 8/03, A61K 8/31, A61Q 17/04, A61Q 19/00, C07C 13/28

(54) **ÖLKÖRPER FÜR KOSMETISCHE ZUSAMMENSETZUNGEN ENTHALTEND CYCLOHEXYLCYCLOHEXAN**
OIL BODIES FOR COSMETIC COMPOSITIONS CONTAINING CYCLOHEXYLCYCLOHEXANE
CORPS HUILEUX POUR PREPARATIONS COSMETIQUES CONTENANT DU CYCLOHEXYLE CYCLOHEXANE

(30) Priorität: 15.03.2002 DE 10211618
(43) Veröffentlichungstag der Anmeldung: 15.12.2004
(73) Patentinhaber: Cognis IP Management GmbH, 40589 Düsseldorf (DE)
(72) Erfinder: KAWA, Rolf, 40789 Monheim (DE); ANSMANN, Achim, 40699 Erkrath (DE); PRINZ, Daniela, 41542 Dormagen (DE); BOTH, Sabine, 40229 Düsseldorf (DE)
(86) Internationale Anmeldenummer: PCT/EP2003/002286
(87) Internationale Veröffentlichungsnummer: WO 2003/077879

(56) Entgegenhaltungen:
- US-A- 4 784 843
- PATENT ABSTRACTS OF JAPAN vol. 013, no. 021 (C-560), 18. Januar 1989 (1989-01-18) & JP 63 225316 A (NITTO ELECTRIC IND CO LTD), 20. September 1988 (1988-09-20)

## Beschreibung

Die Erfindung betrifft neuartige Ölkörper auf Kohlenwasserstoffbasis, die leicht in kosmetische und pharmazeutische Zubereitungen einarbeitbar sind, eine gute dermatologische Verträglichkeit aufweisen und kosmetischen Formulierungen ein besonders leichtes Hautgefühle vermitteln.

Im Bereich kosmetischer Emulsionen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet. Zur Herstellung kosmetischer bzw. pharmazeutischer Zubereitungen werden eine Vielzahl von natürlichen und synthetischen Ölen, beispielsweise Mandel- oder Avocadoöl, Esteröle, Ether, Alkylcarbonate, Kohlenwasserstoffe sowie Silikonöle eingesetzt. Eine wesentliche Aufgabe der Ölkomponenten ist es, neben der pflegenden Wirkung, die in unmittelbarem Zusammenhang mit der Hautfettung steht, dem Konsumenten ein nichtklebriges, möglich rasch eintretendes und lang anhaltendes Gefühl der Hautglätte- und -geschmeidigkeit zu vermitteln.

Das subjektive Empfinden auf der Haut kann mit dem physikochemischen Parameter der Spreitung der Ölkörper auf der Haut korreliert und objektiviert werden, wie dies von U. Zeidler in der Fachzeitschrift Fette, Seifen, Anstrichmittel 87, 403 (1985) dargestellt wurde. Demnach lassen sich kosmetische Ölkörper in niedrigspreitende (< 300 mm²/10 min), mittelspreitende (>1=300 bis <1000 mm²/10 min.) und hochspreitende Öle (>/= 1000 mm²/10 min) einteilen. Setzt man in einer vorgegebenen Formulierung als Ölkörper ein hochspreitendes Öl ein, so wird zwar sehr rasch das gewünschte Gefühl der Hautglättung erzielt und es wird im Falle der Verwendung von Cyclomethiconen, z.B Dow Corning 245 fluid (Dow Corning Corporation) oder Abil B 8839 (Goldschmidt Chemical Coperation) zusätzlich ein vom Verbraucher erwünschtes samtiges Endgefühl gefunden. Dieses Erlebnis dauert jedoch nicht lange an, da durch die hohe Flüchtigkeit der zuletzt genannten Strukturen das ausgeprägte Glättegefühl und damit die Samtigkeit sehr rasch verschwindet und ein unangenehmes stumpfes Gefühl auf der Haut zurück bleibt.

Aufgabe der vorliegenden Erfindung war es, verbesserte, hochspreitende Ölkörper zur Verfügung zu stellen, die ein rasch eintretendes und länger anhaltendes Gefühl der Hautglätte vermitteln und über eine gute Hautverträglichkeit verfügen. Darüber hinaus soll die neue Strukturen einfach und stabil in Emulsionen einzuarbeiten und hydrolyseunempfindlich gegenüber pH - Schwankungen sein.

Gegenstand der Erfindung sind daher kosmetisches Mittel, enthaltend mindestens eine wässerige Phase und eine in der wässerigen Phase nicht lösliche Ölphase, wobei die Ölphase ganz oder teilweise Cyclohexylcyclohexan enthält. Cyclohexylcyclohexan ist eine an sich bekannte Verbindung, die beispielsweise durch basenkatalysierte Aldolkondensation von zwei Teilen Cyclohexanon und anschließender Hydrierung gewonnen werden kann. Das Cyclohexylcyclohexan selbst ist eine farblose Flüssigkeit, die in großer Reinheit (≥ 99 %) hergestellt werden kann. Diese Verbindung ist hochspreitend im Sinne der obigen Definition nach Zeidler (Spreitwerte von mindestens 1600 mm²/10 Minuten).

### Kosmetische Zubereitungen

Die erfindungsgemäße Verbindung erlaubt die Herstellung stabiler kosmetischer Emulsionen. Vorzugsweise handelt es sich hierbei um Formulierungen zur Körperpflege, z. B. in form von Cremes, Milch, Lotionen, sprühbare Emulsionen, Produkte zur Eliminierung des Körpergeruchs etc. Die erfindungsgemäßen Verbindung lässt sich auch in tensidhaltigen Formulierungen wie z. B. Schaum- und Duschbädem, Haarshampoos und Pflegespülungen einsetzen. Vorzugsweise enthalten diese Mittel das Cyclohexylcyclohexan in Mengen von insgesamt 0,1 bis 40 Gew.-%, vorzugsweise in Mengen von 0,1 bis 30 Gew.-%. Die kosmetischen Mittel enthalten Wasser und die Ölphase nebeneinander in form von Emulsionen oder Dispersionen, wobei vorzugsweise W/O oder O/W-Emulsionen bevorzugt sind.

Je nach Applikationszweck enthalten die kosmetischen Formulierungen eine Reihe weiterer Hilfs- und Zusatzstoffe, wie beispielsweise Tenside, weitere Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

### Tenside

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädem, Shampoos etc. ist vorzugsweise wenigstens ein anionisches Tensid enthalten. Der Anteil der Tenside liegt hier üblicherweise bei etwa 1 bis 30, vorzugsweise 5 bis 25 und insbesondere 10 bis 20 Gew.-%.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d. h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Körperpflegemittel, wie Cremes, Lotionen und Milchen, enthalten üblicherweise eine Reihe weiterer Ölkörper und Emollients, die dazu beitragen, die sensorischen Eigenschaften weiter zu optimieren. Die Ölkörper sind üblicherweise in einer Gesamtmenge von 1 - 50 Gew.-%, vorzugsweise 5 - 25 Gew.-% und insbesondere 5 -15 Gew.-% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, lsostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Eru-cyloleat, Erucylbehenat und Erucylerucat. Daneben eignen sich Ester von linearen C₆-C₂₂-Fettsäuren mit verzweigten Alkoholen, insbesondere 2-Ethylhexanol und Isopropanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Feftsäuren, flüssige Mono-/Di-/Triglyceridmischungen auf Basis von C₆-C₁₈₋Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylylcarbonate (Cetiol® CC), Guerbetcarbonate auf Basis von Fettalkoholen mit 6 bis 18, vorzugsweise 8 bis 10 C Atomen, Ester der Benzoesäure mit linearen undloder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäureestem mit Polyolen, Siliconöle (Cyclomethicone, Siliciummethicontypen u.a.) und/oder aliphatische bzw. naphthenische Kohlenwasserstoffe, wie z. B. Mineralöl, Vaseline, Petrolatum, Isohexadecane, Squalan, Squalen oder Dialkylcyclohexane in Betracht.

### Emulgatoren

Als Emulgatoren kommen beispielsweise nicht-ionogene Tenside aus mindestens einer der folgenden Gruppen in Frage:
➢ Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest
➢ Alkyloligoglykoside mit 8 bis 22 Kohlenstoffatomen im Alkylrest und deren ethoxylierte Analoga
➢ Anlagerungsprodukte von 1 bis 15 Mol Ethylenoxid an Ricinusöl und/oder gehärtetes Ricinusöl
➢ Anlagerungsprodukte von 15 bis 60 Mol Ethylenoxid an Ricinusöl und/oder gehärtetes Ricinusöl
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z. B. Sorbit), Alkylglucosiden (z. B. Methylglucosid, Butylglucosid, Laurylglucosid) sowie Polyglucosiden
   (z. B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid
➢ Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin
➢ Mono-, Di- und Trialkylphosphate sowie Mono-, Di- und/oder Tri-PEG-alkylphosphate und deren Salze
➢ Wollwachsalkohole
➢ Polysiloxan-Polyalkyl-Polyether-Copolymere bzw. entsprechende Derivate
➢ Block-Copolymere z. B. Polyethylenglycol-30 Dipolyhydroxystearate
➢ Polymeremulgatoren, z. B. Pemulen-Typen (TR-1,TR-2) von Goodrich
➢ Polyalkylenglycole sowie
➢ Glycerincarbonat.

### ➢ Ethyenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

### ➢ Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische.

Ebenfalls geeignet sind Anlagerungsprodukte von 1 bis 30, vorzugsweise 5 bis 10 Mol Ethylenoxid an die genannten Sorbitanester.

### ➢ Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 lsostearate (Isolan® Gl 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Belina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate lsostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### ➢ Anionische Emulgatoren

Typische anionische Emulgatoren sind aliphatische Fettsäuren mit 12 bis 22 Kohlenstoffatomen wie beispielsweise Palmitinsäure, Stearinsäure oder Behensäure sowie Dicarbonsäuren mit 12 bis 22 Kohlenstoffatomen wie beispielsweise Azelainsäure oder Sebacinsäure.

### ➢ Amphothere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N, N-dimethylammoniumglycinate, beispielsweise das Kokosalkyldimethylammoniumgly-cinat, N-Acylaminopropyl-N,N-dimethylammonium-glycinate, beispielsweise das Kokosacylamino-propyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxy-ethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopro-pionat und das C_{12/18}-Acylsarcosin, Schließlich kommen auch Kationtenside als Emulgatoren in Betracht, wobei solche vom Typ der Esterquats, vorzugsweise methylquaternierte Difettsäure-triethanolaminester-Salze, besonders bevorzugt sind.

### Vorzugsweise enthalten die kosmetischen Zubereitungen

(a) 0,1-30 Gew.-% an Cyclohexylcyclohexan
(b) 0,1- 20 Gew.-% Tenside und/oder Emulgatoren und/oder Coemulgatoren
(c) 0,1- 40 Gew.-% Ölkörper und
(d) 0,1- 98 Gew.-% Wasser bezogen auf die Gesamtzusammensetzung.

### Fette und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste oder flüssige pflanzliche oder tierische Produkte, die im wesentlichen aus gemischten Glycerinestem höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, - palmitat oder-stearat kommen hierfür in Frage. Als Wachse kommen u. a. natürliche Wachse, wie z. B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als weitere Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z. B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z. B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z. B. Magnesium-, Aluminium- und/oder Zinkstearat bzw. -ricinoleat eingesetzt werden.

### Polymere

Geeignete kationische Polymere sind beispielsweise kationische Cellulosederivate, wie z. B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z. B. Luviquat® (BASF), Kondensationsprodukte von Polyglycolen und Aminen, quatemierte Kollagenpolypeptide, wie beispielsweise Lauryldimonium Hydroxypropyl Hydrolyzed Collagen (Lamequat®L/Grünau), quatemierte Weizenpolypeptide, Polyethylenimin, kationische Siliconpolymere, wie z. B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vemetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z. B. Dibrombutan mit Bisdialkylaminen, wie z. B. Bis-Dimethylamino-1,3-propan, kationischer Guar-Gum, wie z. B. Jaguar® CBS, Jaguar® C-17, Jaguar® C-16 der Firma Celanese, quatemierte Ammoniumsalz-Polymere, wie z. B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Iso- bornylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/ Acrylat-Copolymere, Octylacrylamid/Methylmeth-acrylat/tert.Butylaminoethymethacrylat/2-Hydroxypropyl-methacrylat-Copolymere, Polyvinylpyrrolidon, VinylpyrrolidonNinylacetat-Copolymere, Vinylpyrrolidon/ Dimethylaminoethylmethacryla/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkyl-modifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UVB-Filter können öllöslich oder wasserlöslich sein. Als öllösliche Substanzen sind z. B. zu nennen:
➢ 3-Benzylidencampher bzw. 3-Benzylidennorcampher und dessen Derivate, z. B. 3-(4-Methylbenzyliden)campher wie in der EP 0693471 B1 beschrieben
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene)
➢ Ester der Salicylsäure, vorzugsweise Salicylsäure-2-ethylhexylester, Salicylsäure-4-isopropylbenzylester, Salicylsäurehomomenthylester
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
➢ Ester der ßenzalmalonsäure, vorzugsweise 4-Methoxybenzmalonsäuredi-2-ethylhexyl-ester
➢ Triazinderivate, wie z. B. 2,4,6-Trianilino-(p-carbo-2'-ethyl-1'-hexyloxy)-1,3,5-triazin und Octyl-Triazon, wie in der **EP 0818450 A1** beschrieben oder Dioctyl Butamido Triazone (Uvasorb® HEB)
➢ Propan-1,3-dione, wie z. B. 1-(4-tert.Butylphenyl)-3-(4'methoxyphenyl)propan-1,3-dion
➢ Ketotricyclo(5.2.1.0)decan-Derivate, wie in der **EP 0694521 B1** beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze sowie 2,2-(1,4-Phenylene)bis-1H-benzimidazole-4,6 disulfonsäure und deren Salze, insbesondere das Natrium-Salz,
➢ Sulfonsäurederivate von Benzophenonen, vorzugsweise 2-Hydroxy-4-methoxybenzo-phenon-5-sulfonsäure und ihre Salze
➢ Sulfonsäurederivate des 3-Benzylidencamphers, wie z. B. 4-(2-Oxo-3-bomylidenmethyl) benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans, z. B. 4-tert.-Butyl-4'-methoxydibenzoylmethan (Parsol® 1789) und
2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z. B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d. h. hydrophilisiert oder hydrophobiert vorliegen. Typische Beispiele sind gecoatete Titandioxide, wie z. B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Camosin, L-Camosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z. B. ZnO, ZnSO₄) Selen und dessen Derivate (z. B. Selen-Methionin), Stilbene und deren Derivate (z. B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### ➢ Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4- dichlorphenyl)hamstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycetinmonolaurat (GML), Diglycerinmonocaprinat (DMC), Salicylsäure-N-alkylamide wie z. B. Salicylsäure-n-octylamid oder Salicylsäure-n-decylamid.

### ➢ Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure,
Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### ➢ Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzem, Kräutem und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

### ➢ Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe
➢ Ölkomponenten
➢ nichtionische Emulgatoren
➢ Coemulgatoren
➢ Konsistenzgeber
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein.

Solche öllöslichen Hilfsmittel können z. B. sein:
➢ entzündungshemmende, hautschützende oder wohlriechende ätherische Öle
➢ synthetische hautschützende Wirkstoffe und/oder
➢ öllösliche Parfümöle.

Übliche wasserlösliche Zusätze sind z. B. Konservierungsmittel, wasserlösliche Duftstoffe, pH-Wert-Stellmittel, z. B. Puffergemische, wasserlösliche Verdickungsmittel, z. B. wasserlösliche natürliche oder synthetische Polymere wie z. B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazo)-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toiol. 108, 95 (1993)** entnommen werden.

### Insekten-Repellentien

Als Insekten-Repellentien kommen beispielsweise N,N-Diethyl-m-toluamid, 1,2-Pentandiol oder 3-(N-n-Butyl-N-acetyl-amino)-propionic acid-ethyl ester), welches unter der Bezeichnung Insect Repellent® 3535 von der Merck KGaA vertrieben wird, sowie Butylacetylaminopropionate in Frage.

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind:
➢ Glycerin
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton
➢ technische Oligoglyceringemische mit einem Eigenkondensationsgrad von 1,5 bis 10 wie etwa technische Diglyceringemische mit einem Diglyceringehalt von 40 bis 50 Gew.-%
➢ Methyolverbindungen, wie insbesondere Trimethylolethan, Trimethylolpropan, Trimethylolbutan, Pentaerythrit und Dipentaerythrit
➢ Niedrigalkylglucoside, insbesondere solche mit 1 bis 8 Kohlenstoffen im Alkylrest, wie beispielsweise Methyl- und Butylglucosid
➢ Zuckeralkohole mit 5 bis 12 Kohlenstoffatomen, wie beispielsweise Sorbit oder Mannit
➢ Zucker mit 5 bis 12 Kohlenstoffatomen, wie beispielsweise Glucose oder Saccharose
➢ Aminozucker, wie beispielsweise Glucamin
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzyl-salicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone, α-Isomethylionon und Methylcedrylketon, zu den Alkoholen Anethol, Citronellol, Eugenol, Isoeugenol, Geraniol, Linatool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen ver wendet werden. Beispiele sind Kochenillerot A (C.I. 16255), Patentblau V (C.l.42051), Indigotin (C.l.73015), Chlorophyllin (C.l.75810), Chinolingelb (C.l.47005), Titandioxid (C.l.77891) Indanthrenblau RS (C.I. 69800) und Krapplack (C.l.58000) Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

### Beispiele

### Herstellung von Cyclohexylcyclohexan:

2000 g (20,41 mol) Cyclohexanon werden mit 140 g KOH (fest) versetzt. Das Gemisch wird unter Rühren bis unter Rückfluss erhitzt. Nach 0,5 Stunden wird die Reaktion beendet. Das Gemisch wird per GC-Analyse analysiert.

Das Reaktionsgemisch setzt folgendermaßen zusammen:
1. Cyclohexanon: 35,6 %
2. Dimer an α/β-ungesättigtem Keton: 56,9 %.
3. Trimer an α/β-ungesättigtem Keton: 4,5 %
4. Rest: 3 %

Nach dem Erkalten bleibt die KOH zum größten Teil in dem Kolben. Das Gemisch wird mit angesäuertem dest. Wasser (pH-Wert = 3) neutral gewaschen und destillativ von nicht umgesetzten Cyclohexanon, Wasser und Schwersiedem getrennt. Man erhält 720 g Cyclohexanon/Wasser Gemisch als Vorlaufdestillat, 1100 g Dimer Produkt und 170 g Sumpf, der verworfen wird.

### Hydrierung:

### Versuchsapparatur: Hydrierautoklav mit Wasserstoffeinspeisung, Heizvorrichtung

### Versuchsdurchführung:

1000g ungesättigtem α/β-Dimer Keton werden mit 4,4 Gew.-% nickelhaltigem Hydrierkatalysator versetzt. Das Gemisch wird auf 245 °C aufgeheizt und mit Wasserstoff versetzt. Dabei wird 4 Stunden lang ein Wasserstoffdruck von 20 bar angelegt, um abreagierten Wasserstoff zu ersetzen. Nach Beendigung der Reaktion wird das Gemisch auf Raumtemperatur gekühlt. Das so erhaltene Gemisch wird durch fraktionierte Destillation aufgereinigt. Bei 148 °C Sumpftemperatur, 1 mbar Kopfdruck entstehen 5 g Vorlaufdestillat und 980 g Hauptlaufdestillat. Der Sumpf wird verworfen. Der Hauptlauf besteht laut GC-MS Analyse aus 99 % Cyclohexylcyclohexan.

### Anwendungstechnische Untersuchungen:

Die nachfolgenden Beispiele beschreibt die Leistung der erfindungsgemäßen Ölkomponente im Vergleich zu hochspreitenden Öle des Marktes.

Standardemulsion - Mengenangaben in Gew.-% bezogen auf die Endkonzentration

| | |
|---|---|
| Cyclohexylcyclohexan | 20 |
| Eumulgin B2 Ceteareth-2 | 2 |
| Lanette O Cetearyl Alcohol | 2 |
| Glycerin | 5 |
| Carbopol 981 (Carbomer) | 0,2 |
| NaOH pH 7 | pH 7 |
| Wasser, Konservierung | ad 100 |

### Subjektive Beurteilung der Hautglätte

Ein Panel bestehend aus 5 erfahrenen Personen testete Formulierungen auf Basis einer Standardemulsion, die verschiedene hochspreitende Ölkörper enthielt auf ihr subjektives Hautgefühl. Zugrunde gelegt wurde eine Skala zwischen 1 (kaum Glättung, rasche Abnahme des Glättegefühls, niedrige Samtigkeit) und 6 (schnelle, gleichmäßige Glätte, lang anhaltendes Glättegefühl, hohe Samtigkeit). Die Angaben in der Tabelle stellen Mittelwerte dar. Das Beispiel 1 ist erfindungsgemäß, die Beispiele V1 bis V4 dienen dem Vergleich:
1 - erfindungsgemäße Ölkomponente (Spreitwert:1600 mm²/10 min)
V1 - Dow Corning 245 fluid (Spreitwert: >1600 mm²/10 min)
V2 - Abil B 8839 (Spreitwert: >1600 mm²/10 min)
V3 - Crodamol IPM (Isopropyl Myristate) (Spreitwert: 1000 mm²/10 min)
V4 - Cetiol A (Hexyl Laurate) (Spreitwert: 1100mm²/10 min)

| **Beispiel** | **Intensität der Hautglätte** | **Intensität der Samtigkeit** | **Dauer des Erlebnisses** |
|---|---|---|---|
| 1 | 6 | 6 | 6 |
| V1 | 5 | 4 | 2 |
| V2 | 4 | 4 | 2 |
| V3 | 3 | 1 | 1 |
| V4 | 3 | 1 | 1 |

In einer weiteren Untersuchung wurde das Emulgierverhalten der Standardemulsion in Abhängigkeit von den eingesetzten Ölen bewertet. Als Beurteilungskriterium wurde die Größe der Tröpfchen mikroskopisch bewertet und das Emulsionsbild der Prüfemulsion visuell mit einem Standard verglichen, so wie es in Parfümerie und Kosmetik, 80. Jahrgang, Nr. 3/99 Seite 22 dargestellt wurde.
Zugrunde gelegt wurde eine Skala von Tröpfchengröße A: sehr fein bis Tröpfchengröße E: sehr grob. Gleichzeitig wurde die Phasenstabilität nach einer Lagerdauer von 3 Monaten bei 40°C bewertet. Zugrunde gelegt wurde eine Skala von 1: stabil bis 6: Trennung in 2 Phasen

| Beispiel | Tröpfchengröße | Phasenstabilität |
|---|---|---|
| 1 | A | 1 |
| V1 | D | 5 |
| V2 | E | 6 |
| V3 | C | 3 |
| V4 | C | 2 |

Im weiteren sind Beispielrezepturen aufgeführt, welche die vielfältigen Einsatzmöglichkeiten der erfindungsgemäßen Ölphasen demonstrieren.

**Tabelle 1: O/W-Sonnenschutzemulsionen Mengenangaben beziehen sich auf Gew.-% der handelsüblichen Substanzen in der Gesamtzusammensetzung.**

| **Komponente** | **1** | **2** | **3** | **4** | **5** | **6** | **7** | **8** | **9** | **10** | **11** |
|---|---|---|---|---|---|---|---|---|---|---|---|
| **L = Lotion, C = Creme**, | L | C | S | L | C | L | L | C | L | C | L |
| Eumulgin® VL 75 | | | | | | 4 | 4 | 2 | | | |
| Eumulgin® B2 | 2 | | | | | | | | | | |
| **Tween**^{**®**} **60** | | | | 1 | | | | | | | |
| **Myrj**^{**®**} **51** | | 3 | | 2 | | | | | | | |
| Cutina® E 24 | 1 | | | 1 | | | | | | | |
| Hostaphat® KL 340 N | | | | | | | | | 2 | | |
| Lanette® E | | | 0.5 | | | | | 0.5 | | | |
| Amphisol® K | | | 1 | | | 1 | | 0.5 | | 1 | |
| Natriumstearat | | | | | | | 1 | | | | 2 |
| Emulgade® PL 68/50 | | | 1 | | 5 | | | | | 4 | |
| Tego® Care 450 | | | | | | | | | | 3 | |
| Cutina® MD | 2 | | | 6 | | | 4 | | | 6 | |
| Lanette® 14 | 1 | | | 1 | | | | 2 | | | 4 |
| Lanette® O | 1 | 6 | | | 5 | 2 | | 2 | | | |
| Antaron V 216 | | | 1 | | 2 | 2 | | | | 1 | |
| Emery^{®} 1780 | | | | | 0.5 | 0.5 | | | | | |
| Lanolin, wasserfrei USP | | | | | | | 5 | | | | |
| **Cyclohexylcyclohexan** | 2 | 2 | 4 | 1 | 2 | 2 | 2 | 1 | 2 | 2 | 1 |
| Myritol® PC | | | | | 5 | | | | | | |
| Myritol® 331 | 5 | | 8 | | | 6 | | 10 | | 2 | |
| Finsolv® TN | | | 1 | | | | | 1 | 8 | | |
| Cetiol® CC | | 2 | 5 | | | 4 | 4 | 2 | | 2 | |
| Cetiol® OE | | | 3 | | | | | | 2 | 3 | |
| Dow Corning DC® 244 | 4 | | 1 | | 5 | | | 2 | | | 2 |
| Dow Corning DC® 2502 | | 1 | | | 2 | | | | | | |
| Sguatol^{®} S | | | | | | | 4 | | | | |
| Silikonöl Wacker AK^{®} 350 | | 2 | | | | | | | | | |
| Cetiol® 868 | | | | | 2 | | 4 | | | | 7 |
| Cetiol® J 600 | | | | | 3 | 2 | | | | 5 | |
| Mineralöl | | | | 9 | | | | | | | |
| Cetiol® B | | | 1 | | | | | | | 2 | |
| Eutanol® G | | | | | | | | | | | |
| Eutanol® G 16 | | | | | | | | | | | |
| Cetiol® PGL | | 5 | | | | | | | | 5 | |
| Mandelöl | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol /Tocopherylacetat | 1 | | | | | | | | | | |
| Photonyl^{®} LS | | | | | | | | | | | |
| Neo Heliopan® Hydro (Na-Salz) | 2 | | 2.2 | | 3 | 3 | | | | | 2 |
| Neo Heliopan AP (Na-Salz) | 2 | | | | 1,5 | 2 | 2 | | 1 | | 1 |
| Neo Heliopan® 303 | 3 | 5 | 9 | 4 | | | | | | | |
| Neo Heliopan® BB | | | | | 1 | | | | | | 2 |
| Neo Heliopan® MBC | 2 | | | 3 | | 2 | 2 | 2 | | | 1 |
| Neo Heliopan® OS | | | | | | | | | 10 | 7 | |
| Neo Heliopan® E 1000 | | 7.5 | | 6 | | | | | | | 6 |
| Neo Heliopan^{®} AV | | | 7.5 | | | 7.5 | 4 | 5 | | | |
| Uvinul^{®} T 150 | 2 | | | | 2.5 | | | 1 | | | |
| Parsol® 1789 | | 1 | 1 | | | | 2 | | 2 | 2 | |
| Zinkoxid NDM | 10 | | 5 | | | 10 | | 3 | | 5 | 4 |
| Eusolex® T 2000 | | | | | 5 | | 3 | 3 | | | 4 |
| Veegum® Ultra | | | 0.75 | | | | | 1 | 1 | | |
| Keltrol® T | | | 0.25 | | | | | 0.5 | 0.5 | | |
| Carbopol® 980 | | 0.5 | | 0.2 | 0.2 | 0.2 | | 0.5 | 0.1 | 0.3 | 0.2 |
| Ethanol | | | | | | | | | | 10 | |
| Butylenglykol | | 2 | | 4 | 3 | | 2 | 5 | 2 | | 2 |
| Glycerin | 5 | 5 | 5 | | 3 | 3 | 2 | | 4 | | 3 |
| Konservierungsmittel, NaOH | q.s. | | | | | | | | | | |
| Wasser | ad 100 | | | | | | | | | | |

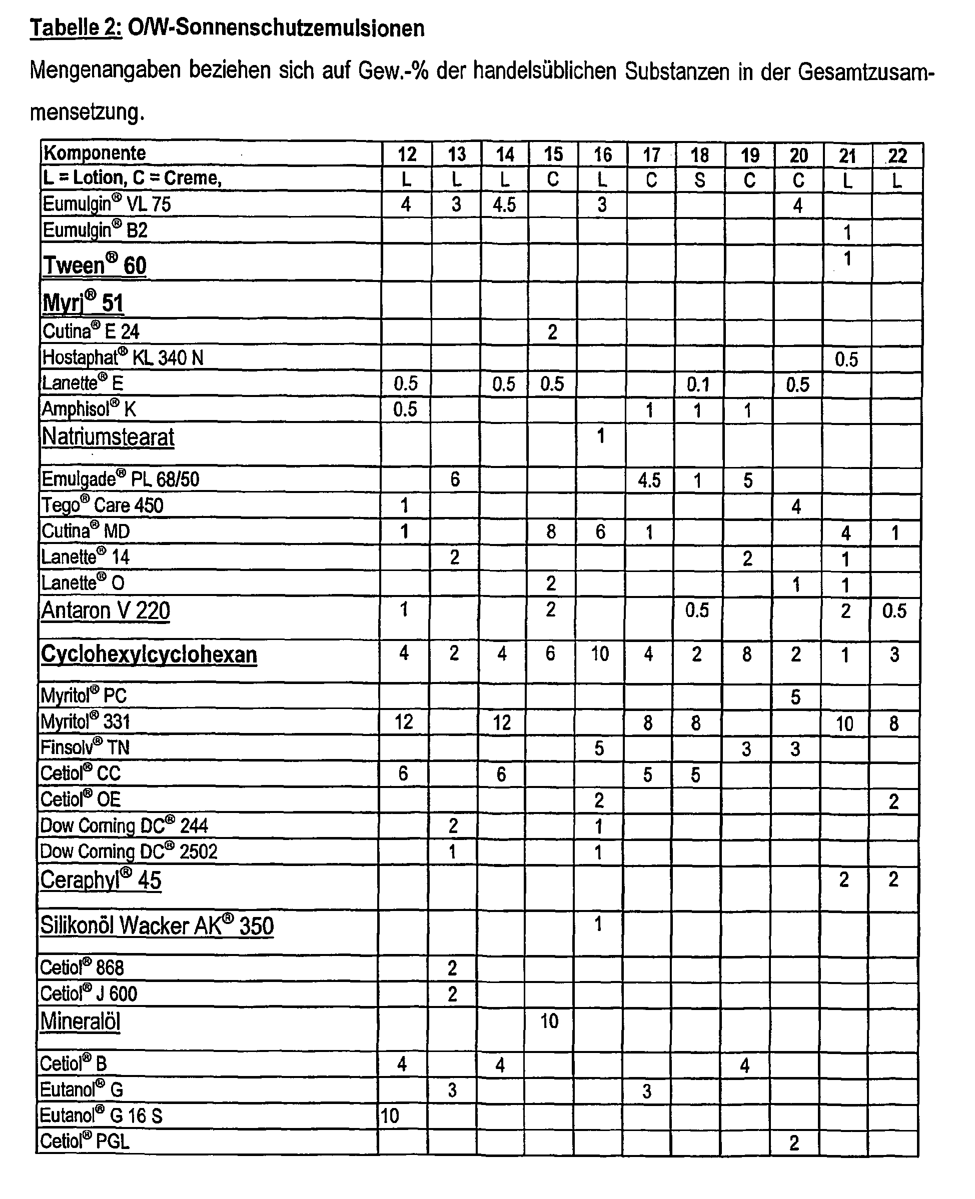

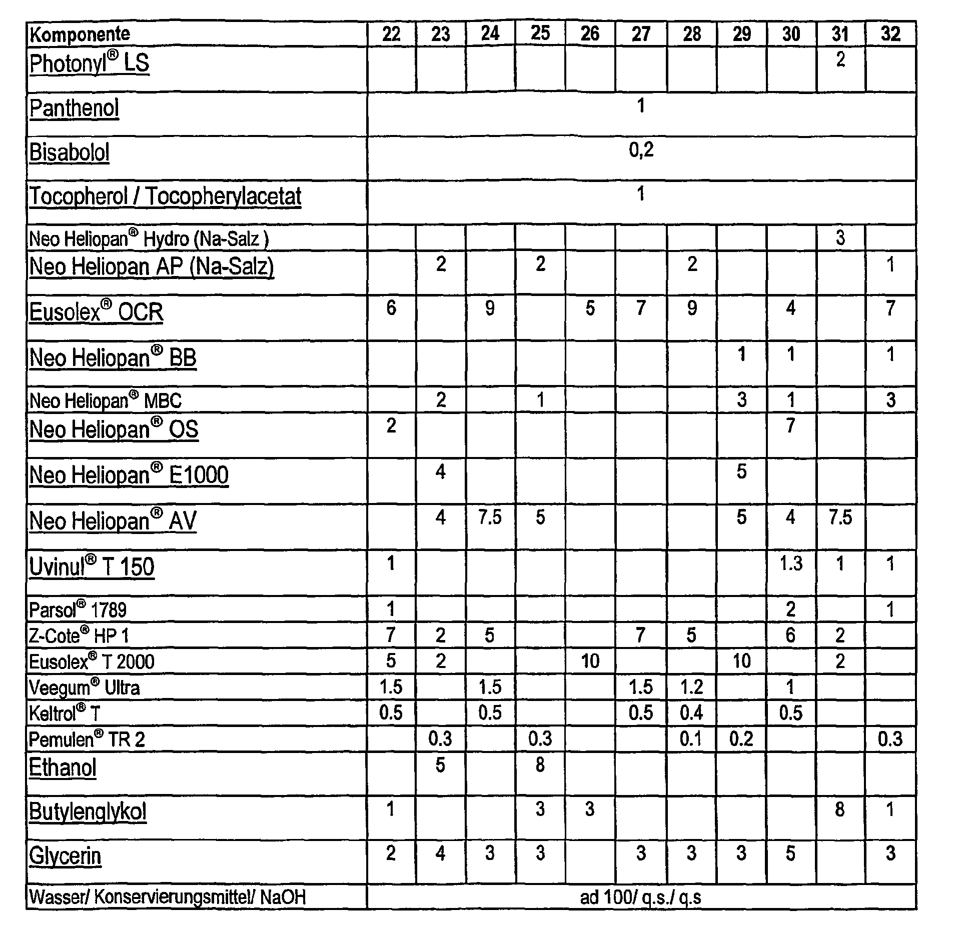

**Tabelle 3: W/O-Sonnenschutzemulsionen Mengenangaben beziehen sich auf Gew.-% der handelsüblichen Substanzen in der Gesamtzusammensetzung.**

| **Komponente** | **33** | **34** | **35** | **36** | **37** | **38** | **39** | **40** | **41** | **42** | **43** |
|---|---|---|---|---|---|---|---|---|---|---|---|
| **L = Lotion; C = Creme** | C | L | C | L | C | L | L | L | L | C | C |
| Dehymuls® PGPH | 4 | 2 | 1 | 3 | 3 | 1 | 1 | 2 | 2 | 4 | 1 |
| Monomuls® 90-O18 | | | 2 | | | | | | | | |
| Lameform® TGI | 2 | | 4 | | 3 | | | | | 1 | 3 |
| Abil® EM 90 | | | | | | | 4 | | | | |
| Glucate® DO | | | | | | | | | | | 3 |
| Isolan® PDI | | | | | | 4 | | 2 | | | |
| Arlacel^{®} 83 | | | | 2 | | | | | | | |
| Elfacos® ST9 | | | | | | | | | 2 | | |
| Elfacos® ST37 | | | | | | | | | | | |
| Arlacel® P 135 | | 2 | | | | | | | | | |
| Dehymuls® HRE 7 | | | | | | | | | | | |
| Zinkstearat | 1 | | | 1 | 1 | | | 1 | | 1 | |
| Microkristallines Wachs | | | 5 | | | 2 | | | | | 5 |
| Bienenwachs | 1 | | | 1 | | | | 5 | | 7 | |
| Tego® Care CG | | | | | 1 | | | | | | .5 |
| Prisorine^{®} 3505 | 1 | | 1 | 1 | | 1 | 1 | | | | 1 |
| Emery^{®} 1780 | | | 5 | | | | | | | 4 | |
| Wollwachsalkohol, wasserfrei, USP | | | | | | | | | | | 1 |
| Antaron V 216 | 2 | | | | | | | | | | |
| **Cyclohexylcyclohexan** | 3 | 4 | 2 | 1 | 10 | 2 | 2 | 6 | 3 | 12 | 1 |
| Myritol® PC | | | | | 3 | | | 4 | | | |
| Myritol® 331 | 10 | | | | 3 | 6 | | | | | 8 |
| Finsolv® TN | | | | 5 | | | 5 | | | | |
| Cetiol® CC | 12 | 22 | | | | 2 | | | 2 | | 5 |
| Cetiol® OE | | | | | 4 | | 5 | | 4 | 2 | |
| Dow Corning DC® 244 | | | | | | | 2 | | | | |
| Dow Corning DC® 2502 | | | 1 | | 2 | | | | | | |
| Prisorine^{®} 3758 | | | | | | | | | | 2 | |
| Silikonöl Wacker AK^{®} 350 | | | | 4 | | | | 3 | | | |
| Cetiol® 868 | | | | | | | | | | 2 | |
| Eutanol® G 16 | | 3 | | | | | | | | | |
| Eutanol® G 16S | | | | | | | | | | | |
| Cetiol® J 600 | | | 4 | | | 2 | | | | | |
| Ceraphyl® 45 | | | | 2 | | | | 2 | | 6 | |
| Mineralöl | | | | | 4 | | | | | | |
| Cetiol® B | | | 2 | 4 | | | | | | 3 | |
| Eutanol® G | | | 3 | | | | | 8 | | | |
| Cetiol® PGL | | 11 | | | | 4 | | | 9 | | |
| Mandelöl | | | | | 1 | | 5 | | | | |
| Photonyl^{®} LS | | 2 | | 1 | | | | | 4 | | |
| Panthenol | 1.0 | | | | | | | | | | |
| Bisabolol | 0.2 | | | | | | | | | | |
| Tocopherol / Tocopherylacetat | 1,0 | | | | | | | | | | |
| Maqnesiumsulfat x 7 Wasser | 1 | | | | | | | | | | |
| Neo Heliopan® Hydro (Na-Salz) | | 2 | | 3 | | | | 2 | | | |
| Neo Heliopan AP (Na-Salz) | 2 | 1 | | | 2 | | | 1 | 2 | | 1 |
| Neo Heliopan^{®} 303 | | | | | 4 | | | | | 6 | |
| Neo Heliopan^{®} BB | | 4 | 2 | | | | 2 | | | | |
| Neo Heliopan® MBC | | | | | | | | 4 | | 3 | |
| Neo Heliopan^{®} OS | | | | | | | | | | | |
| Neo Heliopan^{®} E 1000 | | | | | | | | | 5 | | |
| Neo Heliopan^{®} AV | | 3 | 6 | 6 | | 7.5 | 7.5 | | 5 | | 7.5 |
| Uvinul^{®} T 150 | | | | | 2.5 | | | 1 | | 2 | |
| Parsol^{®} 1789 | | 2 | | | | | | 1 | | 2 | |
| Zinkoxid NDM | | | | | | 6 | | | | | |
| Eusolex® T 2000 | 15 | | 10 | | 5 | | 4 | | | | 4 |
| Ethanol | | | | | | | | | | 8 | |
| Butylenglykol | | | 2 | 6 | | | 2 | 5 | | | 2 |
| Glycerin | 5 | 3 | 3 | | 5 | 3 | 2 | | 10 | 4 | |
| Wasser, Konservierungsmittel | ad 100, q.s. | | | | | | | | | | |

**Tabelle 4: W/O-Sonnenschutzemulsionen Mengenangaben beziehen sich auf Gew.-% der handelsüblichen Substanzen in der Gesamtzusammensetzung.**

| **Komponente** | **44** | **45** | **46** | **47** | **48** | **49** | **50** | **51** | **52** | **53** | **54** |
|---|---|---|---|---|---|---|---|---|---|---|---|
| **L = Lotion; C = Creme** | L | C | L | L | C | L | L | L | L | C | C |
| Dehymuls® PGPH | 3 | 1 | 5 | 1 | 1 | 3 | 2 | 4 | 0.5 | 1 | 4 |
| Monomuls® 90-018 | | 1 | | | | | | | | | |
| Lameform® TGI | | | | | 4 | | | 1 | | 3 | 1 |
| Abil® EM 90 | | | | 1 | | | | | | 2 | |
| Glucate® DO | | | | 3 | | | | | 2 | | |
| Isolan® PDI | | 3 | | | | | 4 | | | | |
| Arlacel^{®} 83 | | | | | | 3 | | | | | |
| Elfacos® ST9 | | | | | | | | | | | 2 |
| Elfacos® ST37 | 2 | | | | | | | | | | |
| Arlacel® P 135 | | | | | | 3 | | | | | |
| Dehymuls® HRE 7 | | | | | | | | | 4 | | |
| Zinkstearat | | 2 | 2 | 1 | 1 | | | 1 | 1 | | |
| Mikrokristallines Wachs | | | | | 4 | | 1 | | | 4 | |
| Bienenwachs | | 4 | | 2 | | | 1 | | 2 | | 1 |
| Tego® Care CG | | | | | | | | | | | |
| Isostearinsäure | 1 | 1 | | | | | 1 | 1 | | 1 | 1 |
| Emery^{®} 1780 | | 7 | 3 | | | | | | | | |
| Wollwachsalkohol, wasserfrei, USP | | | | | | | | | | | |
| Antaron V 220 | | 0.5 | 2 | 1 | 1 | 1 | | | | | |
| Cyclohexylcyclohexan | 2 | 4 | 3 | 3 | 2 | 2 | 1 | 3 | 3 | 1 | 4 |
| Myritol® PC | | | | | | | | | | | |
| Myritol® 331 | 4 | 2 | 3 | | 5 | | | 8 | 5 | 4 | |
| Finsolv® TN | | 5 | 5 | | | 7 | | | | | |
| Cetiol® CC | 3 | 1 | | | | | 3 | 16 | | | 12 |
| Cetiol® OE | | 3 | | 2 | | | 3 | | | | |
| Dow Corning DC® 244 | | 4 | | 2 | | | | | | | |
| Dow Corning DC® 2502 | | | | 1 | | | | | | | |
| Prisorine^{®} 3578 | | 1 | | | | | | | | | |
| Silikonöl Wacker AK^{®} 350 | | | | 1 | | | | | | | |
| Cetiol® 868 | | | | | | | | | | | |
| Eutanol® G 16 | | | | | | | | | | | 3 |
| Eutanol® G 16S | | | | | | | | | | | 7 |
| Cetiol® J 600 | | | | 3 | | | | | | | |
| Ceraphyl® 45 | | | | 1 | | | | | 5 | 4 | |
| Mineralöl | | | | | | | 9 | | | | |
| Cetiol® B | | | | | 3 | 3 | | 2 | 2 | | |
| Eutanol® G | | | | 2 | | | | | 5 | | |
| Cetiol® PGL | | | | | | | | 2 | | | |
| Mandelöl | | | 2 | | | | | | | | |
| Photonyl^{®} LS | | | | | | | 3 | | | | 2 |
| Panthenol | 1.0 | | | | | | | | | | |
| Bisabolol | 0.2 | | | | | | | | | | |
| Tocopherol / Tocopherylacetat | 1.0 | | | | | | | | | | |
| Magnesiumsulfat x 7 Wasser | 1 | | | | | | | | | | |
| Neo Heliopan® Hydro (Na-Salz) | | 4 | | | | | | 4 | | | |
| Neo Heliopan AP (Na-Salz) | 2 | | | 1 | 2 | | 1 | | | | |
| Neo Heliopan^{®} 303 | 6 | 2 | | | | | | | 6 | | |
| Neo Heliopan^{®} BB | | 2 | | 2 | | 2 | | | | | |
| Neo Heliopan® MBC | 2 | | | | 3 | | 4 | | 2 | | |
| Neo Heliopan^{®} OS | | | | | 10 | | 8 | | | | |
| Neo Heliopan^{®} E 1000 | | | 5 | 6 | | | | | | 5 | |
| Neo Heliopan^{®} AV | | 5 | 5 | | | 7.5 | | | | 5 | |
| Uvinul^{®} T 150 | 1 | | | 2 | 2 | | | | 3 | 2 | |
| Parsol^{®} 1789 | | 1 | 1 | | | | 1 | | 0.5 | | |
| Z-Cote® HP 1 | 4 | 10 | | | | | | 5 | | | 5 |
| Titandioxid T 805 | | | | 2 | | 3 | | 7 | | 4 | 7 |
| Ethanol | | | | 8 | | 10 | | | | | |
| Butylenglykol | 5 | 1 | | 3 | 3 | | | | 8 | 2 | |
| Glycerin | | | 6 | 2 | | | 5 | 5 | | 3 | 5 |
| Wasser, Konservierungsmittel | ad 100, q.s. | | | | | | | | | | |

**Tabelle 5: W/O-Pflegeemulsionen Mengenangaben beziehen sich auf Gew.-% der handelsüblichen Substanzen in der Gesamtzusammensetzung.**

| **Komponente** | **55** | **56** | **57** | **58** | **59** | **60** | **61** | **62** | **63** | **64** | **65** |
|---|---|---|---|---|---|---|---|---|---|---|---|
| **L = Lotion. C = Creme** | C | L | C | L | C | L | L | L | C | C | C |
| Dehymuls® PGPH | 1 | 3 | 1 | 2 | 3 | 1 | 1 | 2 | 1 | 1 | 1 |
| Monomuls® 90-O18 | 2 | | | | | | | | 2 | | 2 |
| Lameform® TGI | 4 | 1 | | | 3 | | | 1 | 4 | 3 | 3 |
| Abil® EM 90 | | | | | | | 4 | | | | |
| Isolan® PDI | | | | | | 4 | | | | | |
| Glucate® DO | | | | 5 | | | | | | | |
| Arlacel^{®} 83 | | | 5 | | | | | | | | |
| Dehymuls® FCE | | | | | | | | | | | |
| Dehymuls® HRE 7 | | | | | | | | 4 | | 1 | |
| Zinkstearat | 2 | 1 | | 1 | 1 | | | 1 | 1 | 1 | |
| Mikrokristallines Wachs | | | 5 | | | 2 | | | | | 5 |
| Bienenwachs | 4 | | | 1 | | | | 1 | 4 | 7 | |
| Tego Care® CG | | | | | 1 | | | | | | 0.5 |
| Prisorine^{®} 3505 | | | 1 | 1 | | 1 | 1 | | | | 1 |
| Dry Flo® Plus | | | | | | | | | | | |
| SFE 839 | | | | | | | 3 | | | | |
| Emery^{®} 1780 | 1 | | | | | | | | | | 1 |
| Lanolin; anhydrous USP | | | 5 | | | | | | | 4 | |
| **Cyclohexylcyclohexan** | 3 | 4 | 2 | 12 | 10 | 2 | 2 | 6 | 3 | 12 | 1 |
| Cegesoft® C 17 | | | 3 | | | | | | | 1 | |
| Myritol® PC | | | | | | 2 | | 4 | | | |
| Myritol® 331 | 6 | | | | 2 | 6 | 2 | | | | 8 |
| Finsolv® TN | | | | 5 | | 2 | 5 | | | | |
| Cetiol® A | | 6 | | | | 4 | | | | | |
| Cetiol® CC | | 8 | | | 2 | 2 | 2 | | | | 5 |
| Cetiol® SN | | 5 | | | | | | 3 | | | |
| Cetiol® OE | 3 | | | | 4 | | 2 | | 4 | 2 | |
| Dow Corning DC® 244 | | | | | 1 | | 2 | | | | |
| Dow Corning DC® 2502 | | | 1 | | 2 | | | | | | |
| Prisorine^{®} 3758 | | | | | 3 | | | | | | |
| Silikonöl Wacker AK® 350 | | | | 4 | | | | 3 | | | |
| Cetiol® 868 | | | | | | | | | | 2 | 7 |
| Cetiol® J 600 | | | 4 | | | 2 | | | | | |
| Ceraphyl® 45 | | | | 2 | | | | 2 | | 6 | |
| Mineralöl | | | | | 4 | | | | | | |
| Cetiol® B | | | 2 | 4 | | | | | | 3 | |
| Eutanol® G 16 | | 1 | | | | | | | | 3 | |
| Eutanol® G | | | 3 | | | | | 8 | | | |
| Cetiol® PGL | | | | | | 4 | | | 9 | | |
| Mandelöl | | | | | 1 | | 5 | | | | |
| Insect Repellent^{®} 3535 | 2 | | | | | | | | | | |
| N,N-Diethyl-m-toluamid | | | | 3 | | | | 5 | | | |
| Photonyl^{®} LS | 2 | 2 | | | | | | | | | |
| Panthenol | 1.0 | | | | | | | | | | |
| Bisabolol | 0.2 | | | | | | | | | | |
| T**ocopherol /Tocopheryl Acetate** | 1.0 | | | | | | | | | | |
| Magnesiumsulfat x 7 Wasser | 1 | | | | | | | | | | |
| Bentone^{®} 38 | | | | | 1 | | | | | | |
| Propylencarbonat | | | | | 0.5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Wasser, Konservierungsmittel | Ad 100, q.s. | | | | | | | | | | |

**Tabelle 6: W/O-Pflegeemulsionen Mengenangaben beziehen sich auf Gew.-% der handelsüblichen Substanzen in der Gesamtzusammensetzung.**

| **Komponente** | **66** | **67** | **68** | **69** | **70** | **71** | **72** | **73** | **74** | **75** | **76** |
|---|---|---|---|---|---|---|---|---|---|---|---|
| **L = Lotion. C = Creme** | L | C | L | L | C | L | L | L | L | C | C |
| Dehymuls® PGPH | 3 | 1 | 5 | 1 | 1 | 3 | 3 | 4 | 1 | 1 | 1 |
| Monomuls® 90-O18 | | 1 | | 1 | | | | | | | |
| Lameform® TGI | | | | | 4 | | | 1 | | 3 | |
| Abil® EM 90 | | | | 3 | | | | | | 2 | |
| Isolan® PDI | | 3 | | | | | | | | | 4 |
| Glucate® DO | 1 | | | | | | | | | | |
| Arlacel^{®} 83 | | | | | | 3 | | | | | |
| Dehymuls® FCE | | | | | 4 | | 1 | | | | |
| Dehymuls® HRE 7 | | | | | | | | | 7 | | |
| Zinkstearat | | 2 | 2 | 1 | 1 | 1 | | 1 | 1 | | 1 |
| Mikrokristallines Wachs | | | | | 4 | | 1 | | | 4 | |
| Bienenwachs | | 4 | | 2 | | 2 | 1 | 1 | 2 | | 5 |
| Tego® Care CG | | | | | | | | | | | |
| Prisorine^{®} 3505 | 1 | 1 | | | | | 1 | 1 | | 1 | 1 |
| Dry Flo® Plus | 1 | | | | | | | | | | |
| SFE® 839 | | 5 | | | | 4 | | | | | |
| Emery^{®} 1780 | | | | | | | | | | | |
| Lanolin anhydrous USP | | 7 | 3 | | | | | | | | |
| **Cyclohexylcyclohexan** | 3 | 4 | 4 | 8 | 10 | 2 | 8 | 6 | 3 | 12 | 7 |
| Cegesoft® C 17 | | 2 | | | | | | | | | |
| Myritol® PC | | | | 8 | | | | | | | |
| Myritol® 331 | 4 | | 3 | | 5 | 3 | | | 5 | 4 | |
| Finsolv® TN | | | 5 | | | 7 | | | | | |
| Cetiol® A | | | | | | | | 6 | | | |
| Cetiol® CC | 3 | | | 6 | | 3 | 3 | | | 8 | |
| Cetiol® SN | | | | | 5 | | | | | | |
| Cetiol® OE | | 3 | | 2 | | | 3 | | | | 8 |
| Dow Corning® DC 244 | | 4 | | 2 | | 2 | | | | | |
| Dow Corning® DC 2502 | | | | 1 | | | | | | | |
| Prisorine^{®} 3758 | | | | | | 1 | | | | | |
| Silikonöl Wacker AK® 350 | | | | 1 | | 1 | | 4 | | | |
| Cetiol® 868 | | | | | | | | | | | 10 |
| Cetiol® J 600 | 4 | | | 3 | | | | | | | |
| Ceraphyl® 45 | | | | 1 | | | | | 5 | 4 | |
| Mineralöl | | | | | | | 9 | | | | |
| Cetiol® B | | | | | 3 | 3 | | 2 | 2 | | |
| Eutanol® G 16 | 1 | | | | | | | | | | |
| Eutanol® G | | | | 2 | | | | | 5 | | |
| Cetiol® PGL | | | 10 | | | | | 6 | | | 3 |
| Mandelöl | | | 2 | | 5 | | 2 | | | | |
| Photonyl^{®} LS | | | | 2 | | | | | | | 2 |
| Panthenol | 1.0 | | | | | | | | | | |
| Bisabolol | 0.2 | | | | | | | | | | |
| **Tocopherol /Tocopherylacetat** | 1.0 | | | | | | | | | | |
| Magnesiumsulfat x 7 Wasser | 1 | | | | | | | | | | |
| Bentone^{®} 38 | | | | | | 1 | | | | | |
| Propylencarbonat | | | | | | 0.5 | | | | | |
| Ethanol | | | | 8 | | 10 | | | | | |
| Butylenalykol | 5 | 1 | | 3 | 3 | | | | 8 | 2 | 1 |
| Glycerin | | | 6 | 2 | | | 5 | 5 | | 3 | 5 |
| Wasser, Konservierungsmittel | ad 100, q.s. | | | | | | | | | | |

**Tabelle 7: O/W-Pflegeemulsionen Mengenangaben beziehen sich auf Gew.-% der handelsüblichen Substanzen in der Gesamtzusammensetzung.**

| **Komponente** | **77** | **78** | **79** | **80** | **81** | **82** | **83** | **84** | **85** | **86** | **87** |
|---|---|---|---|---|---|---|---|---|---|---|---|
| **L = Lotion, C = Creme** | C | C | C | L | C | L | L | C | L | C | C |
| Eumulgin® VL 75 | | | | | | 4 | | | | | |
| Dehymuls® PGPH | | 2 | | | | | | | | | |
| Generol® R | | | 1 | | | | | | | | |
| Eumulgin® B2 | | | 0.8 | | | | | | | | |
| **Tween**^{**®**} **60** | | | | 1 | | | | | | | |
| Cutina® E 24 | | | 0.6 | 2 | | | | | | | |
| Hostaphat® KL 340 N | | | | | | | | | 2 | | |
| Lanette® E | | | | | | | | 1 | | | |
| Amphisol® K | | 0.5 | | | | 1 | | | | 1 | 0.5 |
| Natriumstearat | | | | | 0.5 | | | | | | |
| Emulgade® PL 68/50 | | 2.5 | | | | | | | | 4 | |
| Tego® Care CG | | | | | | | | | | | 2 |
| Tego® Care 450 | | | | | | | | 5 | | | |
| Cutina® MD | | 1 | | 6 | 5 | | 4 | | | 6 | |
| Lanette® 14 | | | | 1 | | | | 2 | | | 4 |
| Lanette® O | 4.5 | | 4 | | 1 | 2 | | | | | 2 |
| Novata® AB | | 1 | | | | | | | | | 1 |
| Emery^{®} 1780 | | | | | 0.5 | 0.5 | | | | | |
| Lanolin, wasserfrei, USP | | | | | | | 5 | | | | |
| Cetiol® SB 45 | | | 1.5 | | | | 2 | | | | |
| **Cyclohexylcyclohexan** | 3 | 4 | 2 | 1 | 10 | 2 | 2 | 6 | 3 | 12 | 1 |
| Cegesoft® C 17 | | | | | | | | | | | |
| myritol® PC | | | | | 5 | | | | | | |
| Myritol® 331 | 2 | 5 | 5 | | | 6 | | 12 | | | |
| Finsolv® TN | | | 2 | | | 2 | | | 8 | | |
| Cetiol® CC | 4 | 6 | | | | 4 | 4 | | | | 5 |
| Cetiol® OE | | | | | | | | | 4 | 3 | |
| Dow Corning DC® 245 | | | 2 | | 5 | 1 | | | | | |
| Dow Corning DC® 2502 | | | | | 2 | 1 | | | | | |
| Prisorine^{®} 3758 | | | | | | 1 | | | | | |
| Silikonöl Wacker AK^{®} 350 | 0.5 | 0.5 | 0.5 | | | 1 | 4 | | | | |
| Cetiol® 868 | | | | | 2 | | 4 | | | | |
| Cetiol® J600 | 2 | | 3 | | 3 | 2 | | | | 5 | |
| Ceraphyl® 45 | | | | | | | 3 | | | | |
| Mineralöl | | | | 9 | | | | | | | |
| Cefiol® SN | | | 5 | | | | | | | | |
| Cetiol® B | | | | | | | | | | 2 | |
| Eutanol® G | | 2 | | 3 | | | | | | | |
| Cetiol® PGL | | | | | | | | | 5 | 5 | |
| Dry Flo ® Plus | 5 | | | | | | 1 | | | | |
| SFE 839 | 5 | | | | | | | | | | 2 |
| Mandelöl | | | | | | | 1 | | | | |
| Insect Repellent^{®} 3535 | | 2 | 4 | | | 2 | | | | 3 | |
| N,N-Diethyl-m-toluamid | | 2 | | | | | | | | 3 | |
| Photonyl® LS | 2 | 2 | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum® ultra | | | | | | | | | 1 | | |
| Keltrol® T | | | 0.4 | | | | | | 0.5 | | |
| Pemulen® TR 2 | 0.3 | | | | | | | 0.3 | | | |
| Carbopol® Ultrez 10 | | 0.3 | 0.3 | 0.2 | 0.2 | 0.2 | | | 0.1 | 0.3 | 0.2 |
| Ethanol | | | | | | | | | | 10 | |
| Butylenuglkol | | | | 4 | 3 | | 2 | 5 | 2 | | |
| Glycerin | 2 | 5 | 5 | | 3 | 3 | 2 | | 4 | | 3 |
| Wasser, Konservierungsmittel, NaOH | ad 100, q.s., | | | | | | | | | | |
| | pH 6,5 - 7,5 | | | | | | | | | | |

**Tabelle 8: O/W-Pflegeemulsionen Mengenangaben beziehen sich auf Gew.-% der handelsüblichen Substanzen in der Gesamtzusammensetzung.**

| **Komponente** | **88** | **89** | **90** | **91** | **92** | **93** | **94** | **95** | **96** | **97** | **98** |
|---|---|---|---|---|---|---|---|---|---|---|---|
| **L = Lotion, C = Creme** | C | C | L | C | L | C | L | L | L | L | C |
| Eumulgin® VL 75 | 4 | 3 | | | | | 1 | | | | 2 |
| Generol® R | | | | | | 2 | | | | | |
| Eumulgin® B2 | | | | | | 2 | | | | 1 | |
| **Tween**^{**®**} **60** | | | | | | | | | | 1 | |
| Cutina® E 24 | | | | 2 | | | | | | | |
| Hostaphat® KL 340 N | | | | | | | | | | | |
| Lanette® E | 0.5 | | | | | | | | | | 1 |
| Amphisol® K | 0.5 | 1 | | | | | | 1 | 1 | | |
| Natriumstearat | | | | | 1 | | | | | | |
| Emulgade® PL 68/50 | | 6 | | | | | | 5 | | | 4 |
| Tego® Care CG | | | | | | | | | | | |
| Tego® Care 450 | | | | | | | | | 4 | | |
| Cutina® MD | 3 | | 3 | 8 | 6 | 8 | | | | 4 | |
| Lanette® 14 | | 2 | | | | | | 2 | | 1 | |
| Lanette® O | 2 | | | 2 | | 3 | 1 | | 1 | 1 | 6 |
| Novata® AB | | | | | | | | | | | |
| Emery^{®} 1780 | | | | | | | | | | | |
| Lanolin, wasserfrei, USP | | | | | | 4 | | | | | |
| Cetiol® SB 45 | | | | | | | 2 | | | | |
| **Cyclohexylcyclohexan** | 3 | 4 | 2 | 1 | 10 | 2 | 2 | 6 | 3 | 12 | 1 |
| Cegesoft® C 17 | 4 | | | | | | | | | | |
| Myritol® PC | 6 | | | | | 5 | | | 5 | | |
| Myritol® 331 | 5 | | 5 | | | | 7 | | | 10 | 3 |
| Finsolv® TN | | 5 | | | 5 | | | 3 | 3 | | 1 |
| Cetiol® CC | | | | | | | | | | | 2 |
| Cetiol® OE | | | | | 2 | | 2 | | 5 | | |
| Dow Corning DC® 245 | | 2 | | | 1 | | | | | 8 | 2 |
| Dow Corning DC® 2502 | | 1 | | | 1 | | | | | | 3 |
| Prisorine^{®} 3758 | 3 | | | | | | | | | | 2 |
| Silikonöl Wacker AK^{®} 350 | | | | | 1 | | | | | | 1 |
| Cetiol® 868 | | 2 | | | | | | | | | |
| Cetiol® J 600 | | 2 | | | | | | | | | |
| Ceraphyl® 45 | | | | | | | 3 | | | | |
| Cetiol® SN | | | | | | | | | | | |
| Cetiol® B | | | 5 | | | 5 | | 4 | | | 3 |
| Eutanol® G | | 3 | 5 | | 5 | | | | | | |
| Cetiol® PGL | | | | | | | | 5 | 2 | | |
| Dry Flo® Plus | | 1 | | | | | | | | | 1 |
| SFE 839 | 1 | 1 | | | | | | | | | |
| Mandelöl | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopherylacetat | 1 | | | | | | | | | | |
| Veegum® Ultra | | | | | | | | | 1 | | |
| Keltrol® T | | | | | | | | | 0.5 | | |
| Carbopol® ETD 2001 | | 0.3 | | 0.3 | | 0.5 | 0.2 | 0.2 | | | |
| Pemulen^{®} TR 2 | | | 0.3 | | | 0.3 | | | | | 0.5 |
| Ethanol | | 5 | | 8 | | | | | | | 10 |
| Butylenglykol | 5 | | 2 | 3 | 3 | | | | | 8 | |
| Glycerin | 2 | 4 | 3 | 3 | | 7 | 5 | 3 | 5 | | |
| Wasser, Konservierungsmittel, NaOH | ad 100, q.s. | | | | | | | | | | |
| | (pH 6,5-7,5) | | | | | | | | | | |

**Tabelle 9: Sprayformulierungen Mengenangaben beziehen sich auf Gew.-% der handelsüblichen Substanzen in der Gesamtzusammensetzung.**

| **Komponente** | **99** | **100** | **101** | **102** | **103** | **104** | **105** | **106** | **107** | **108** | **109** |
|---|---|---|---|---|---|---|---|---|---|---|---|
| **S = Körperspray, S*= = Sonnenschutzspray** | S | S | S | S | S | S* | S* | S* | S* | S* | S* |
| Emulgade® SE-PF | 8,9 | | 7,5 | 7.5 | 4,3 | 9.8 | 8.2 | 9.9 | | | |
| Eumulgin® B2 | 3,1 | | 3 | | | | | 4.2 | | | |
| **Eumulgin**^{**®**} **B3** | | | | | | 4.2 | 3.3 | | | | |
| **Eumulgin**^{**®**} **HRE 40** | | | | | 4,7 | | | | | | |
| Cutina® E 24 | | 5,9 | | 4 | | | | | | | |
| Amphisol® K | | | | | | | | | 1 | 1 | 1 |
| Eumulgin® VL 75 | | | | | | | | | | | 2 |
| Emulgade® PL 68/50 | | 0.5 | | | | | | | 2.5 | 1 | |
| Cutina® MD | | 3,1 | | | | | | | | | |
| Antaron V 220 | | | | | | 1 | 1 | 1 | | 1 | 1 |
| **Cyclohexylcyclohexan** | 11 | 5 | 7 | 7 | 7 | 5 | 4 | 5 | 5 | 4 | 6 |
| Myritol® PC | | | | | | | | | | | |
| Myritol® 331 | | | 3 | 4 | 3 | 3 | 3 | 3 | | | |
| Finsolv® TN | | 4 | | | | | | 8 | | | |
| Cetiol® CC | 6 | | | 5 | 5 | 2 | 2 | 4 | | | |
| Cetiol® OE | | 5 | 5 | | | 2 | | | | | |
| Dow Corning DC® 244 | | 4 | 4 | 5 | | | | | | | |
| Cetiol® 868 | 3 | | | | | | | | | | |
| Cetiol® J 600 | | | | 2 | 2 | | | | | | |
| Mineralöl | | | 2 | | | | | | | | |
| Cetiol® B | | | | | | | 2 | | | | |
| Eutanol® G | 2 | | | | 1 | | | | | | |
| Photonyl^{®} LS | 2 | | | | | | 2 | | | 2 | 2 |
| Panthenol | 1 | | | | | | | | | | |
| Bisabolol | 0,2 | | | | | | | | | | |
| Tocopherol / Tocopherylacetat | 1 | | | | | | | | | | |
| Neo Heliopan® Hydro (Na-Salz) | | | | | | 2 | | | | 3 | |
| Neo Heliopan AP (Na-Salz) | | | | | | 2 | 2 | 2 | | | 1 |
| Eusolex^{®} OCR | | | | | | | 2 | | | | 3 |
| Neo Heliopan^{®} BB | | | | | | | | | | 1 | |
| Neo Heliopan® MBC | | | | | | 2 | 2 | 2 | | 1 | 1 |
| Neo Heliopan^{®} OS | | | | | | 5 | | | | | |
| Neo Hetiopan^{®} AV | | | | | | 6 | 6 | 2 | | 7.5 | 2 |
| Uvinul^{®} T 150 | | | | | | 1 | 1 | 1 | | 1 | |
| Parsol® 1789 | | | | | | 1 | | 1 | | 1 | |
| Z-cote® HP 1 | | | | | | | | | | 2 | 2 |
| Eusolex® T 2000 | | | | | | | | | | 2 | 2 |
| Veegum® Ultra | | | | | | | | | | | 1.5 |
| Laponite® XLG | | | | | | | | | | 1.5 | |
| Keltrol® T | | | | | | | | | | | 0.5 |
| Pemulen® TR 2 | | | | | | | | | 0.2 | | |
| Insect Repellent^{®} 3535 | 1 | | | | | | | | | | |
| N,N-Diethyl-m-toluamid | 1 | | | | | | | | | | |
| Ethanol | | | | | | | | | | | |
| Butylenglykol | | | | | | | 1 | | | 2 | 1 |
| Glycerin | | | | | | 3 | 2 | 3 | 2 | | 3 |
| Wasser/ Konservierungsmittel/ NaOH | ad 100/ q.sJ q.s | | | | | | | | | | |

### Anhang

| | |
|---|---|
| | 14) Cetiol® 868 |
| 1) Abil® EM 90 | INCI: Ethylhexyl Stearate |
| INCI: Cetyl Dimethicone Copolyol | Hersteller: Cognis Deutschland GmbH |
| Hersteller: Tego Cosmetics (Goldschmidt) | |
| | 15) Cetiol® A |
| 2) Amphisol® K | INCI: Hexyl Laurate |
| INCl: Potassium Cetyl Phosphate | Hersteller: Cognis Deutschland GmbH |
| Hersteller: Hoffmann La Roche | |
| | 16) Cetiol® B |
| 3) Antaron® V 220 | INCI: Butyl Adipate |
| INCl: PVP/Eicosene Copolymer | Hersteller: Cognis Deutschland GmbH (Henkel) |
| Hersteller: GAF General Aniline Firm Corp. (IPS-Global) | |
| | 17) Cetiol® J 600 |
| 4) Antaron® V 216 | INCl: Oleyl Erucate |
| INCI: PVP/Hexadecene Copolymer | Hersteller: Cognis Deutschland GmbH |
| Hersteller: GAF General Aniline Firm Corp. (IPS-Global) | |
| | 18) Cetiol® OE |
| 5) Arlacel® 83 | INCI: Dicaprylyl Ether |
| INCI: Sorbitan Sesquioleate | Hersteller: Cognis Deutschland GmbH |
| Hersteller Uniqema (ICI Surfacants) | |
| | 19) Cetiol® PGL |
| 6) Arlacel® P 135 | INCI: Hexyldecanol, Hexyldecyl Laurate |
| INCI: PEG-30 Dipolyhydroxystearate | Herstellen Cognis Deutschland GmbH |
| Hersteller. Uniqema (ICI Surfacants) | |
| | 20) Cetiol® CC |
| 7) Bentone® 38 | INCl: Dicaprylyl Carbonate |
| INCl: Quaternium-18 Hectorite | Hersteller: Cognis Deutschland GmbH |
| Hersteller: Rheox (Elementis Specialties) | |
| | 21) Cetiol® SB 45 |
| 8) Carbopol® 980 | INCI: Shea Butter Butyrospermum Parkii (Linne) |
| INCl: Carbomer | Herstellen Cognis Deutschland GmbH |
| Hersteller: Goodrich | |
| | 22) Cetiol® SN |
| | INCl: Cetearyl Isononanoate |
| 9) Carbopol® 2984 | Hersteller: Cognis Deutschland GmbH (Henkel) |
| INCl: Carbomer | |
| Hersteller. Goodrich | 23) Cutina® E 24 |
| | INCl: PEG-20 Glyceryl Stearate |
| 10) Carbopol® ETD 2001 | Hersteller: Cognis Deutschland GmbH |
| INCl: Carbomer | |
| Hersteller: BF Goodrich | 24) Cutina® MD |
| | INCl: Glyceryl Stearate |
| 11) Carbopol® Ultrez 10 | Hersteller: Cognis Deutschland GmbH |
| INCl: Carbomer | |
| Hersteller: Goodrich | 25) Dehymul® FCE |
| | INCl: Dicocoyl Pentaerythrityl Distearyl Citrate |
| 12) Cegesott® C 17 | Hersteller: Cognis Deutschland GmbH |
| INCl: Myristyl Lactate | |
| Hersteller: Cognis Deutschland GmbH, Grünau | 26) Dehymuls® HRE 7 |
| | INCl: PEG-7 Hydrogenated Castor Oil |
| 13) Ceraphyl® 45 | Hersteller: Cognis Deutschland GmbH |
| INCl: Diethylhexyl Malate | |
| Hersteller: International Specialty Products | 27) Dehymuls® PGPH |
| | INCl: Polyglyceryl-2 Dipolyhydroxystearate |
| | Hersteller: Cognis Deutschland GmbH |
| | 42) Eutanol® G 16 |
| 28) Dow Corning® 244 Fluid | INCI: Hexyldecanol |
| INCI: Cyclomethicone | Hersteller: Cognis Deutschland GmbH |
| Hersteller: Dow Corning | |
| | 43) Eutanol® G 16 S |
| 29) Dow Corning® 245 Fluid | INCI: Hexyldecyl Stearate |
| INCI: Cyclopentasiloxane Cyclomethicone | Hersteller. Cognis Deutschland GmbH |
| Hersteller: Dow Corning | |
| | 44) Finsolv® TN |
| 30) Dow Corning® 2502 | INCI: C 12/15 Alkyl Benzoate |
| INCI: Cetyl Dimethicone | Hersteller: Findex (Nordmann/Rassmann) |
| Hersteller: Dow Corning | |
| | 45) Generol® R |
| 31) Dry® Flo Plus | INCI: Brassica Campestris (Rapseed) Sterols |
| INCI: Aluminium Starch Octenylsuccinate | Hersteller: Cognis Deutschland GmbH |
| Hersteller: National Starch | |
| | 46) Glucate® DO |
| 32) Elfacos® ST 37 | INCl: Methyl Glucose Dioleate |
| INCl: PEG-22 Dodecyl Glycol Copolymer | Hersteller: NRC Nordmann/Rassmann |
| Hersteller: Akzo-Nobel | |
| | 47) Hostaphat® KL 340 N |
| 33) Elfacos® ST 9 | INCl: Trilaureth -4 Phosphate |
| INCl: PEG-45 Dodecyl Glycol Copolymer | Hersteller: Clariant |
| Hersteller: Akzo-Nobel | |
| | 48) Isolan® PDI |
| 34) Emery® 1780 | INCl: Diisostearoyl Polyglyceryl-3 Diisosteara |
| INCl: Lanolin Alcohol | Hersteller: Goldschmidt AG |
| Hersteller: Cognis Corporation (Emery) | |
| | 49) Keltrol® T |
| 35) Emulgade® PL 68/50 | INCl: Xanthan Gum |
| INCl: Cetearyl Glucoside, Ceteayl Alcohol | Hersteller: CP Kelco |
| Hersteller: Cognis Deutschland GmbH | |
| | 50) Lameform® TGI |
| 36) Emulgade® SE-PF | INCI: Polyglyceryl-3 Diisostearate |
| INCl: Glyceryl Stearate, Ceteareth-20, Ceteareth-12, | Hersteller: Cognis Deutschland GmbH |
| Cetearyl Alcohol, Cetyl Palmitate | |
| Hersteller: Cognis Deutschland GmbH | 50) Lanette® 14 |
| | INCl: Myristyl Alcohol |
| 37) Eumulgin® B 2 | Hersteller. Cognis Deutschland GmbH |
| INCl: Ceteareth- 20 | |
| Hersteller: Cognis Deutschland GmbH | 51) Lanette® E |
| | INCl: Sodium Cetearyl Sulfate |
| 38) Eumulgin® VL 75 | Hersteller: Cognis Deutschland GmbH |
| INCl: Lauryl Glucoside (and) Polyglyceryl-2 Dipolyhydro- | |
| xystearate (and) Glycerin | 52) Lanette® O |
| Hersteller: Cognis Deutschland GmbH | INCl: Cetearyl Alcohol |
| | Hersteller: Cognis Deutschland GmbH |
| 39) Eusolex® OCR | |
| INCI: Octocrylene | 53) Monomuls® 90-0-18 |
| Hersteller: Merck | INCI: Glyceryl Oleate |
| | Hersteller: Cognis Deutschland GmbH |
| 40) Eusolex® T 2000 | |
| INCI: Titanium Dioxide, Alumina, Simethicone | 54) Myrj® 51 |
| Hersteller: Rona (Merck) | INCI: PEG-30-Sterate |
| | Hersteller: Uniqema |
| 41) Eutanol® G | |
| INCl: Octyldodecanol | 55) Myritol® 331 |
| Hersteller: Cognis Deutschland GmbH | INCl: Cocoglycerides |
| | Hersteller: Cognis Deutschland GmbH |
| 56) Myritol® PC | 70) Prisorine® 3758 |
| INCI: Propylene Glycol Dicaprylate/Dicaprate | INCI: Hydrogenated Polyisobutene |
| Hersteller: Cognis Deutschland GmbH | Hersteller: Uniqema |
| | |
| 57) Neo Heliopan® 303 | 71) Ravecarb® 106 |
| INCl: Octocrylene | Polycarbonatdiol |
| Hersteller: Haarmann & Reimer | Hersteller: Enichem |
| | |
| 58) Neo Heliopan® AP | 73) SFE® 839 |
| INCl: Disodium Phenyl Dibenzimidazole Tetrasulfonate | INCl: Cyclopentasiloxane and Dimethicone/Vinyl |
| Hersteller: Haarmann & Reimer | Dimethicone Crosspolymer |
| 59) Neo Heliopan® AV | Hersteller: GE Silicones |
| INCl: Ethylhexyl Methoxycinnamate | |
| Hersteller: Haarmann & Reimer | 74) Silikonöl Wacker AK® 350 |
| | INCl: Dimethicone |
| 60) Neo Heliopan® BB | Hersteller. Wacker |
| INCl: Benzophenone-3 | |
| Hersteller. Haarmann & Reimer | 75) Squatol® S |
| | INCl: Hydrogenated Polyisobutene |
| 61) Neo Heliopan® E 1000 | Hersteller: LCW (7-9 rue de l'Industrie 95310 St-Ouen |
| INCl: Isoamyl-p-Methoxycinnamate | l'Aumone France) |
| Hersteller: Haarmann & Reimer | |
| | 76) Tego® Care 450 |
| 62) Neo Heliopan® Hydro (Na-Salz) | INCI: Polyglyceryl-3 Methylglucose Distearate |
| INCI: Phenylbenzimidazole Sulfonic Acid | Hersteller: Tego Cosmetics (Goldschmidt) |
| Hersteller: Haarmann & Reimer | |
| | 77) Tego® Care CG 90 |
| 63) Neo Heliopan® MBC | INCI: Cetearyl Glucoside |
| INCI: 4-Methylbenzylidene Camphor | Hersteller: Goldschmidt |
| Hersteller: Haarmann & Reimer | |
| | 78) Tween® 60 |
| 64) Neo Heliopan® OS | INCI: Polysorbate 60 |
| INCI: Ethylhexyl Salicylate | Hersteller: Uniqema (ICI Surfactants) |
| Hersteller: Haarmann & Reimer | |
| | 79) Uvinul® T 150 |
| 65) Novata® AB | INCI: Octyl Triazone |
| INCI: Cocoglycerides | Hersteller: BASF |
| Hersteller: Cognis Deutschland GmbH | |
| | 80) Veegum® Ultra |
| 66) Parsol® 1789 | INCl: Magnesium Aluminium Silicate |
| INCl: Butyl Methoxydibenzoylmethane | Hersteller. Vanderbilt |
| Hersteller. Hoffmann-La Roche (Givaudan) | |
| | 81) Z-Cote® HP 1 |
| 67) Pemulen® TR-2 | INCI: Zinc Oxide, Dimethicone |
| INCl: Acrylates /C10-30 Alkylacrylate Crosspo- | Hersteller: BASF |
| lymer | |
| Hersteller. Goodrich | |
| | |
| 68) Photonyl® LS | |
| INCl: Arginine, Disodium Adenosine | |
| Triphosphate, Mannitol, Pyridoxine HCL, Phenylalanine, | |
| Tyrosine | |
| Hersteller Laboratoires Serobiologiques (Cognis) | |
| | |
| 69) Prisorine® ISAC 3505 | |
| INCl: Isostearic Acid | |
| Hersteller: Uniqema | |

## Patentansprüche

1. Kosmetisches Mittel, enthaltend mindestens eine wässerige Phase und eine in der wässerigen Phase nicht lösliche Ölphase, **dadurch gekennzeichnet, daß** die Ölphase ganz oder teilweise Cyclohexylcyclohexan enthält.

2. Kosmetisches Mittel nach Anspruch 1, **dadurch gekennzeichnet, daß** es 90 bis 10 Gew.-% Wasser und 10 bis 90 Gew.-% der Ölphase enthält.

3. Kosmetisches Mittel nach den Ansprüchen 1 und 2, **dadurch gekennzeichnet, daß** es zwischen 0,1 und 40 Gew.-% an Cyclohexylcyclohexan enthält.

4. Kosmetisches Mittel nach den Ansprüchen 1 bis 3, **dadurch gekennzeichnet, daß** es in Form einer W/O- oder O/W-Emulsion vorliegt.

5. Kosmetisches Mittel nach den Ansprüchen 1 bis 4, **dadurch gekennzeichnet, daß** es enthält
(a) 0,1- 30 Gew.-% an Cyclohexylcyclohexan
(b) 0,1- 20 Gew.-% Tenside und/oder Emulgatoren und/oder Coemulgatoren
(c) 0,1 - 40 Gew.-% Ölkörper und
(d) 0,1- 98 Gew.-% Wasser bezogen auf die Gesamtzusammensetzung.

6. Verwendung von Cyclohexylcyclohexan als Ölphase in wässerigen kosmetischen Mitteln.

## Claims

1. A cosmetic preparation cotaining at least one aqueous phase and an oil phase insoluble in the aqueous phase, **characterized in that** the oil phase completely or partly contains cyclohexyl cyclohexane.

2. A cosmetic preparation as claimed in claim 1, **characterized in that** it contains 90 to 10% by weight water and 10 to 90% by weight oil phase.

3. A cosmetic preparation as claimed in claims 1 and 2, **characterized in that** it contains between 0.1 and 40% by weight cyclohexyl cyclohexane.

4. A cosmetic preparation as claimed in claims 1 to 3, **characterized in that** it is present in the form of a w/o or o/w emulsion.

5. A cosmetic preparation as claimed in claims 1 to 4, **characterized in that** it contains
(a) 0.1 to 30% by weight cyclohexyl cyclohexane,
(b) 0.1 to 20% by weight surfactants and/or emulsifiers and/or co-emulsifiers,
(c) 0.1 to 40% by weight oil components and
(d) 0.1 to 98% by weight of water,
based on the composition as a whole.

6. The use of cyclohexyl cyclohexane as an oil phase in water-based cosmetic preparations.

## Revendications

1. Produit cosmétique, contenant au moins une phase aqueuse et une phase huileuse non soluble dans la phase aqueuse,
**caractérisé en ce que**
la phase huileuse est constituée entièrement ou en partie de cyclohexylcyclohexane.

2. Produit cosmétique selon la revendication 1,
**caractérisé en ce qu'**
il contient de 90 à 10 % en poids d'eau et de 10 à 90 % en poids de phase huileuse.

3. Produit cosmétique selon les revendications 1 et 2,
**caractérisé en ce qu'**
il contient entre 0,1 et 40 % en poids de cyclohexylcyclohexane.

4. Produit cosmétique selon les revendications 1 à 3,
**caractérisé en ce qu'**
il se présente sous la forme d'une émulsion eau dans l'huile E/H ou huile dans l'eau H/E.

5. Produit cosmétique selon les revendications 1 à 4,
**caractérisé en ce qu'**
il contient
(a) de 0,1 à 30 % en poids de cyclohexylcyclohexane,
(b) de 0,1 à 20 % en poids de tensioactifs et/ou d'émulsifiants et/ou de co-émulsifiants,
(c) de 0,1 à 40 % en poids de corps huileux et
(d) de 0,1 à 98 % en poids d'eau par rapport à la composition totale.

6. Utilisation de cyclohexylcyclohexane en tant que phase huileuse dans des produits cosmétiques aqueux.
